# EUROPEAN PATENT APPLICATION

(11) **EP 2 455 100 A2**
(43) Date of publication of application: **23.05.2012**
(21) Application number: 11009926.4
(22) Date of filing: 27.10.2006
(51) Int. Cl.: A61K 39/00, A61K 39/395, G01N 33/53

(54) **Reagents, methods and systems for selecting a cytotoxic antibody or variant thereof**

(30) Priority: 07.11.2005 US 73419605 P
(62) Divisional of application: 06826738.4
(71) Applicant: The Rockfeller University, New York, NY 10021-6399 (US)
(72) Inventor: Ravetch, Jeffrey, New York, NY 10022 (US); Nimmerjahn, Falk, 91054 Erlangen (DE)
(74) Representative: Schmidt, Karsten

(57) **Abstract**

The present invention provides reagents, methods and systems for predicting the cytotoxic activity of an antibody or variant thereof comprising: determining a binding affinity of the antibody or variant thereof to a Fc activating receptor; determining a binding affinity of the antibody or variant thereof to a Fc inhibitory receptor, and calculating the ratio of said activating binding affinity to said inhibitory binding affinity (A/I ratio), wherein the magnitude of said ratio is an indication of the cytotoxic activity of the antibody or variant thereof . The present invention also provides purified modified antibodies having altered A/I ratios as compared to the unmodified antibodies.

## Description

### CROSS REFERENCE TO RELATED APPLICATIONS

This application claims the benefit of provisional application number 60/734,196, filed on November 7, 2005, which is incorporated herein by reference.

### STATEMENT REGARDING FEDERALLY FUNDED RESEARCH

The Research Leading to the present invention was supported in part, by National Institutes of Health Grant No. CA 80757. Accordingly, the U.S. Government may have certain rights in this invention.

### FIELD OF THE INVENTION

The present invention relates to a novel method for designing therapeutic antibodies and vaccines for treatment of microbial infection, cancer and autoimmune disease.

### BACKGROUND OF THE INVENTION

The mammalian immune system has evolved to defend the organism against pathogenic microbes, layering the specificity of adaptive responses on the ancestral pathways of innate immunity. This complexity exists to provide discrimination between self and non-self and to insure that immune responses are tightly regulated, thus avoiding autotoxicity and uncontrolled inflammation. Multiple checkpoints have been identified that function to insure an orderly progression through an immune response and thereby prevent the generation of self destructive processes. A common theme that has emerged from the study of these checkpoints is the requirement for the establishment of discrete thresholds that define narrow windows of response. One mechanism to achieve these thresholds is for the co-expression of receptors with common ligand binding properties but divergent signaling capacities, coupling activating receptors with an inhibitory counterpart thereby setting thresholds for immune cell activation (Ravetch, Fc receptors. In Fundamental Immunology, W. E. Paul, ed. (Philadelphia, Lippincott-Raven), pp- 685-700 (2003)).

Immune complexes (IC) consisting of IgG antibodies have long been recognized to have potent immunoregulatory functions ranging from a strong enhancement to complete suppression of antibody responses (reviewed in Heyman, Annu Rev Immunol 18, 709-737 (2000)), in addition to their more overt roles as effector molecules for the elimination of foreign antigens. These divergent activities of IgGs can now be explained through the selective engagement of specific FcγRs on discrete cell types, which result in either arrest or progression of an immune response, determined by the specific checkpoint and threshold achieved.

Although cellular receptors for immunoglobulins were first identified nearly 40 years ago, their central role in the immune response was only discovered in the last decade. They are key players in both the afferent and efferent phase of an immune response, setting thresholds for B cell activation and antibody production, regulating the maturation of dendritic cells and coupling the exquisite specificity of the antibody response to effector pathways, such as phagocytosis, antibody dependent cellular cytotoxicity and the recruitment and activation of inflammatory cells. Their central role in linking the humoral immune system to innate effector cells has made them attractive immunotherapeutic targets for either enhancing or restricting the activity of antibodies *in vivo*.

The interaction of antibodies and antibody-antigen complexes with cells of the immune system effects a variety of responses, including antibody dependent cell-mediated cytotoxicity (ADCC) and complement dependent cytotoxicity (CDC), phagocytosis, inflammatory mediator release, clearance of antigen, and antibody half-life (reviewed in Daron, Annu Rev Immunol, 15, 203-234 (1997); Ward and Ghetie, Therapeutic Immunol, 2, 77-94 (1995); Ravetch and Kinet, Annu Rev Immunol, 9, 457-492 (1991)), each of which is incorporated herein by reference).

Antibody constant domains are not involved directly in binding an antibody to an antigen, but exhibit various effector functions. Depending on the amino acid sequence of the constant region of their heavy chains, antibodies or immunoglobulins can be assigned to different classes. There are five major classes of immunoglobulins: IgA, IgD, IgE, IgG, and IgM, and several of these may be further divided into subclasses (isotypes), e.g., IgG1, IgG2, IgG3, and IgG4; IgA1 and IgA2. The heavy chain constant regions that correspond to the different classes of immunoglobulins are called α, δ, ε, γ, and µ, respectively. Of the various human immunoglobulin classes, human IgG1 and IgG3 mediate ADCC more effectively than IgG2 and IgG4.

Papain digestion of antibodies produces two identical antigen binding fragments, called Fab fragments, each with a single antigen binding site, and a residual "Fc" fragment, whose name reflects its ability to crystallize readily. The Fc region is central to the effector functions of antibodies. The crystal structure of the human IgG Fc region has been determined (Deisenhofer, Biochemistry, 20, 2361-2370 (1981), which is incorporated herein by reference). In human IgG molecules, the Fc region is generated by papain cleavage N-terminal to Cys, 226.

Several antibody functions are mediated by Fc receptors (FcRs), which bind the Fc region of an antibody. FcRs are defined by their specificity for immunoglobulin isotypes: Fc receptors for IgG antibodies are referred to as FcγR, for IgE as FcεFR, for IgA as FcαR and so on. Surface receptors for immunoglobulin G are present in two distinct classesthose that activate cells upon their crosslinking ("activation FcRs") and those that inhibit activation upon co-engagement ("inhibitory FcRs").

In all mammalian species studied to date, four different classes of Fc-receptors have been defined: FcγRI (CD64), FcγRII (CD32), FcγRIII (CDI6) and FcγRIV. Whereas FcγRI displays high affinity for the antibody constant region and restricted isotype specificity, FcγRII and FcγRIII have low affinity for the Fc region of IgG but a broader isotype binding pattern (Ravetch and Kinet, 1991; Hulett and Hogarth, Adv Immunol 57, 1-127 (1994)). FcγRIV is a recently identified receptor, conserved in all mammalian species with intermediate affinity and restricted subclass specificity (Mechetina et al., Immunogenetics 54, 463-468 (2002); Davis et al., Immunol Rev 190, 123-136 (2002) ; Nimmerjahn et al., Immunity 23, 41-51 (2005)).

Functionally there are two different classes of Fc-receptors: the activation and the inhibitory receptors, which transmit their signals via immunoreceptor tyrosine based activation (ITAM) or inhibitory motifs (ITIM), respectively (Ravetch, in Fundamental Immunology W. E. Paul, Ed. (Lippincott-Raven, Philadelphia, (2003); Ravetch and Lanier, Science 290, 84-89 (2000). The paired expression of activating and inhibitory molecules on the same cell is the key for the generation of a balanced immune response. Additionally, it has only recently been appreciated that the IgG Fc-receptors show significant differences in their affinity for individual antibody isotypes rendering certain isotypes more strictly regulated than others (Nimmerjahn et al., 2005).

The mouse expresses three activation FcγRs, FcRI, FcRIII and FcRIV, oligomeric surface receptors with a ligand binding α subunit and an ITAM containing γ subunit. The inhibitory receptor is FcγRIIB, a single chain receptor with an ITIM sequence found in the cytoplasmic tail of the ligand binding α chain. FcRIIB and FcRIII bind monomeric IgG with an affinity constant of 1x10⁶; hence, under physiological conditions they do not bind monomeric IgG, but interact with multimeric IgG immune complexes with low affinity and high avidity. FcRIII and FcRIV are physiologically important activation FcRs for mediating inflammatory disease triggered by cytotoxic antibodies or pathogenic immune complexes. FcRIII is expressed on dendritic cells, NK cells, macrophages, monocytes, mast cells and neutrophils in the mouse, while FcRIV is found on dendritic cells, macrophages, monocytes and neutrophils. They are not found on B cells, T cells, red blood cells or platelets. FcRIIB is found on most hematopoeitic cells, including dendritic cells, B cells, macrophages, monocytes mast cells and neutrophils. It is not found on T cells or NK cells. FcRII and III have greater than 90% sequence identity in their extracellular, ligand binding domain, while FcRIV is most homologous to human FcRIIIA

The situation in the human is analogous. There are three low-affinity activation FcRs for IgG-FcγRIIA, FcγRIIC and FcγRIIIA, FcγRIIA, and FcγRIIC are a single-chain low affinity receptors for IgG, with an ITAM sequence located in their cytoplasmic tail. They are expressed on dendritic cells, macrophages, mast cells, monocytes, neutrophils and some B cells. They are 90% homologous in their extracellular domains to the human inhibitory FcRIIB molecule, which has an ITIM sequence in its cytoplasmic domain, expressed on dendritic cells, B cells, macrophages, mast cells, neutrophils, monocytes but not NK cells or T cells. FcRIIIA is an oligomeric activation receptor consisting of a ligand binding subunit and an ITAM containing γ our ζ subunit. It is expressed on NK cells, macrophages and mast cells. It is not expressed on neutrophils, B cells or T cells. In addition, a receptor with greater than 95% sequence identity in its extracellular domain called FcRIIIB is found on human neutrophils as a GPI-anchored protein. It is capable of binding immune complexes but not activating cells in the absence of association with an ITAM containing receptor like FcRIIA. FcRII and FcRIII are about 70% identical in their ligand binding extracellular domains.

Thus, in the human, IgG cytotoxic antibodies interact with four distinct low-affinity receptors--three of which are capable of activating cellular responses, FcRIIA, FcRIIC and FcRIIIA, one of which is inhibitory, FcRIIB and one of which will bind IgG complexes but not trigger cellular responses, FcRIIIB. Macrophages expresses FcRIIA, FcRIIB and FcRIIIA, neutrophils express FcRIIA, FcRIIB and FcRIIIB, while NK cells express only FcRIIIA. The efficacy of a therapeutic anti-tumor antibody will thus depend on the specific interactions with activation, inhibition and inert low-affinity FcRs, differentially expressed on distinct cell types.

Diversification of IgG subclasses is most strikingly observed in mammals where detailed characterization of four subclasses has been described (Litman et al., Annu Rev Immunol 17, 109-47 (1999)). In both rodents and primates these subclasses display differential abilities to mediate effector responses, such as antibody dependent cytotoxicity, phagocytosis and release of inflammatory mediators (Burton and Woof, Adv Immunol 51, 1-84 (1992); Ravetch, (2003) pp. 685-700; Ravetch and Bolland, Annu Rev Immunol 19, 275-90 (2001)). Skewing of the expression of IgG subclasses is regulated by both the antigen and cytokine milieu, such that IL-4 preferentially induces switching to IgG1 and IgE, while TGF-β induces switching to IgG2b and IgA (Finkelman et al., Annu Rev Immunol 8, 303-33. (1990); Stavnezer, J Immunol 155, 1647-51 (1995); Snapper and Mond, Immunol Today 14, 15-7 (1993)). Thymic dependent antigens primarily result in IgG1, 2a and 2b responses; in contrast, thymic independent antigens typically lead to IgG3 accumulation (Mond et al., Curr Opin Immunol 7, 349-54 (1995)). Further distinctions among the subclasses occurs in response to T cell derived responses with T_{H1} cytokines resulting in IgG2a, 2b and 3 switching, while T_{H2} cytokines lead to IgG1 and IgE dominated responses (Mosmann, and Coftman, Annu Rev Immunol 7, 145-73 (1989)). Among the IgG subclasses, IgG2a and 2b are generally considered to be the most potent at activating effector responses and have been found to dominate in both anti-viral and autoimmune conditions (Coutelier et al., J Exp Med 165, 64-9 (1987); Markine-Goriaynoff and Coutelier, J Virol 76, 432-5. (2002); Fossati-Jimack et al., J Exp Med 191, 1293-302 (2000); Uchida et al., J Exp Med 199, 1659-69 (2004)). The reasons for such functional distinction have not been previously determined until the studies described herein.

In addition, multiple mechanisms have been proposed for the ability of anti-tumor antibodies to mediate their effects *in vivo,* including extended half-life, blockade of signaling pathways, activation of apoptosis and effector cell mediated cytotoxicity. The elucidation of a mechanism that enhances the ability of anti-tumor antibodies to effectively treat tumors is highly desirable. Accordingly, there is an immediate need for improved reagents, methods and systems for designing therapeutic antibodies and vaccines for treatment of microbial infection, cancer and autoimmune disease.

### SUMMARY OF THE INVENTION

The present invention fills the foregoing need by providing an advantageous strategy for enhancing effector function of therapeutic antibodies, particular anti-tumor, anti-viral, and anti-microbial (bacteria and unicellular parasites) antibodies, in mammals including humans.

The present invention represents an important improvement over prior art efforts to regulate antibody mediated immune responses. For example, Shields (PCT 00/42072, J Biol Chem 276, 6591-6604 (2001)) does not distinguish among the antibody isotypes and activation FcRs, nor provide a means of predicting *in vivo* activity. Similarly, Lazar (US Patent Application 20040132101) does not provide a general method for predicting *in vivo* activity and provides no guidance for distinguishing among antibody variants. The present invention, by recognizing that the cytotoxic activity of an antibody or variant thereof can be predicted, provides a method in which one of skill in the art can by: (a) determining a binding affinity of the antibody to Fc activating receptors, (b) determining a binding affinity of the antibody to a Fc inhibitory receptor, and (c) calculating the ratio (A/I ratio) of said activating binding affinity to said inhibitory binding affinity, predict the *in vivo* activity of an antibody or variant wherein the magnitude of said ratio is an indication of the cytotoxic activity of the antibody and predictive of its *in vivo* activity against a tumor, viral or microbial target. One aspect of the present invention provides a general and widely applicable method of selecting a cytotoxic antibody or variant thereof out of a plurality of antibodies comprising: comparing the A/I ratios of the plurality of antibodies; and selecting the cytotoxic antibody or variant thereof with the highest A/I ratio, up to a maximal value of 500, to avoid monomeric engagement of an antibody with its receptor, thus rendering it unable to mediate crosslinking of activation receptors required to trigger effector responses.

Another aspect of the present invention provides a method of selecting one or more antibodies or variants thereof with reduced cytotoxic activity or perhaps "non-cytotoxic" activity comprising determining the A/I ratio of a plurality of antibodies or variants thereof and selecting those antibodies or variants thereof with a A/I ratio of less than 1.

Another aspect of the present invention provides a method of selecting one or more cytotoxic antibodies or variants thereof comprising determining the A/I ratio of a plurality of cytotoxic antibodies or variants thereof and selecting those cytotoxic antibodies or variants thereof with a A/I ratio of 1 or greater. A preferred aspect of the present invention provides a method of selecting one or more cytotoxic antibodies or variants thereof comprising determining the A/I ratio of a plurality of cytotoxic antibodies or variants thereof and selecting those cytotoxic antibodies or variants thereof with a A/I ratio of between about 1 to less than 500.

In some embodiments, the cytotoxic antibody is a human cytotoxic antibody. In this embodiment of the invention the Fc activating receptor is selected from the group consisting of FcγRII and FcγRIII, and an Fc inhibitory receptor is FcγRIIB. A preferred embodiment of the present invention provides that the human FcγRII activating receptor is FcγRIIA or FcγRIIC, wherein the human FcγRIII activating receptor is FcγRIIIA, and wherein the human FcγRII inhibitory receptor is FcγRIIB.

Another embodiment of the present invention provides that the cytotoxic antibody is a chimeric antibody. Another embodiment of the present invention provides that the cytotoxic antibody is a humanized antibody.

In some embodiments, the A/I ratio is determined by one or more quantitative assays, including competition or sandwich ELISA, a radioimmunoassay, a dot blot assay, a fluorescence polarization assay, a scintillation proximity assay, a homogeneous time resolved fluorescence assay, a resonant mirror biosensor analysis, and a surface plasmon resonance analysis. In the competition or sandwich ELISA, the radioimmunoassay, or the dot blot assay the A/I ratio can be determined by coupling the assay with a statistical analysis method, such as, for example, Scatchard analysis. Scatchard analysis is widely known and accepted in the art and is described in, for example, Munson et al., Anal Biochem, 107:220 (1980).

Another aspect of the present invention provides a purified modified antibody comprising an IgG Fc region, the modified antibody having a greater A/I ratio as compared to the unmodified antibody. In yet another aspect of the present invention provides a purified modified antibody comprising an IgG Fc region, the modified antibody having a lower A/I ratio as compared to the unmodified antibody.

In some embodiments, the purified modified antibody comprises a human IgG1, IgG2, IgG3 or IgG4 Fc region, wherein the modified antibody binds to a human Fc activating receptor selected from the group consisting of FcγRII and FcγRIII, and wherein the modified antibody binds to a human FcγRII inhibitory receptor. One embodiment of the invention provides that the modified antibody has an increased cytotoxicity activity *in vitro*. A preferred embodiment of the present invention provides that the human FcγRII activating receptor is FcγRIIa or FcγRIIc, wherein the human FcγRIII activating receptor is FcγRIIIa, and wherein the human FcγRII inhibitory receptor is FcγRIIb.

In another embodiment of the present invention the purified modified antibody has an altered amount of sialic acid compared to an unaltered antibody.

In another embodiment of the present invention the purified modified antibody has a lower amount of sialic acid compared to an unmodified antibody. In this embodiment of the invention, the purified modified antibody is either derived from a naturally occurring antibody or is expressed in a cell line having a protein sialylation deficiency.

These and other aspects of the invention will be better understood by reference to the Drawings, Detailed Description, and Examples.

### BRIEF DESCRIPTION OF THE DRAWINGS

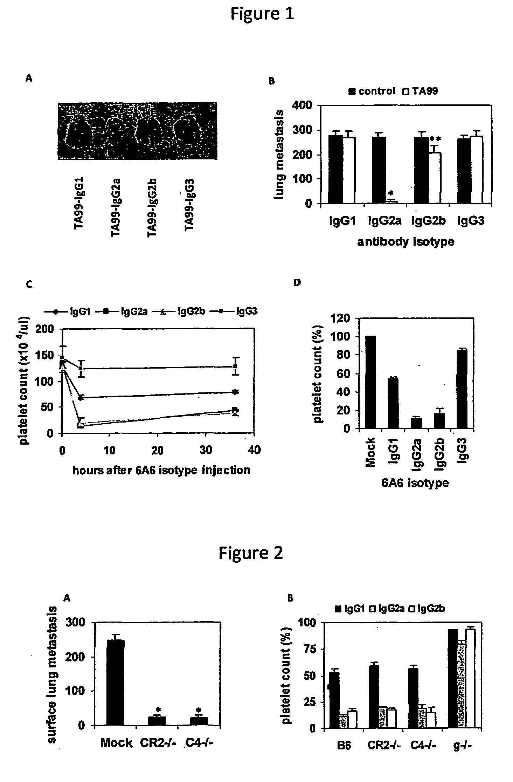

Fig. 1 illustrates the hierarchy of antibody-isotype mediated effector functions *in vivo.* Shown are the B16-F10 lung metastasis and platelet depletion models in C57BL/6 mice (mean ±SEM). (A and B) Mice were injected with B16-F10 melanoma cells followed by injection of TA99-isotype switch variants or control antibodies (200 µg per injection) on days 0, 2, 4, 7, 9 and 11. Mice were sacrificed 15 days after tumor cell injection and the number of surface lung metastasis was evaluated. Asterisk indicates P < 0.0001; double asterisks *P* < 0.01. (C and D) Mice were injected with 4 µg of 6A6-antibody switch variants and platelet counts were determined at the indicated time points (C). (D) The platelet count 4 hours after injection of the 6A6 isotype variants is shown as the percentage of the total platelet count before antibody injection.
Fig. 2 illustrates that IgG2a-mediated effects are independent of the complement cascade *in vivo* (mean+/-SEM). (A) Mice deficient for complement receptor 2 (CR2-/-) or complement component C3 or C4 (C4-/-) were injected with B16-F10 melanoma cells and treated with 100 µg of the TA99-IgG2a antibody per injection. After 15 days animals were sacrificed and lung surface metastasis count was determined. Asterisk indicates *P* < 0.0001. B) C57BL/6, γ-chain-/-, CR2-/-, and C4-/- mice were injected with 4 µg of the 6A6-IgGl, -IgG2a, or IgG2b antibody isotypes and platelet counts were determined before and 4 hours after antibody injection (Nimmerjahn, et al., (2005). Shown is the platelet count 4 hours after antibody injection relative to the platelet count before injection percent. The experiments were done twice with 3-4 animals per group.

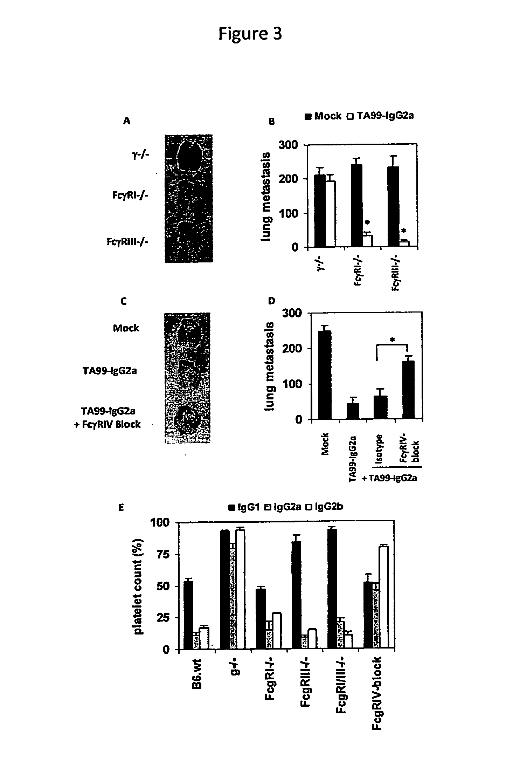

Fig. 3 illustrates the Fcγ receptor dependence of antibody isotype-mediated effector functions (A and B). Mice deficient for the common γ-chain (γ-/-), or α chains of activation Fcγ-receptor I (FcγRI-/-) or III (FcγRIII-/-) were injected with B16-F10 melanoma cells and treated with 100 µg of the TA99-IgG2a antibody or PBS (mock) as described. At day 15 after tumor cell injection mice were sacrificed, lungs were prepared (A) and the number of lung surface metastasis was quantified (B) (mean+/-SEM). Asterisk indicates *P* < 0.0001. The experiment was performed twice with 5 mice per group. (C and D) FcγRI-/- mice were injected with B16-F10 melanoma cells and treated with the TA99-IgG2a antibody at 100 µg per injection. At days 0, 2 and 4 after tumor cell injections mice were injected with 200 µg of an FcγRIV blocking antibody or an isotype matched control antibody. Lungs were prepared at day 15 after tumor cell injection (C) and lung surface metastasis were quantified (D) (mean+/-SEM). Asterisk indicates P<0.001. (E) The indicated mouse strains were injected with 4 µg of the 6A6-isotype switch variants and the platelet count was determined 4 hours after injection of the respective antibody variants (mean+/-SEM). To block immune complex binding to FcγRIV mice were injected with 200 µg of an FcγRIV-blocking antibody (Nimmerjahn, F., et al., (2005)). Shown is the relative platelet count 4 hours after antibody injection in percent. Experiments were performed twice with 4-6 mice per group.

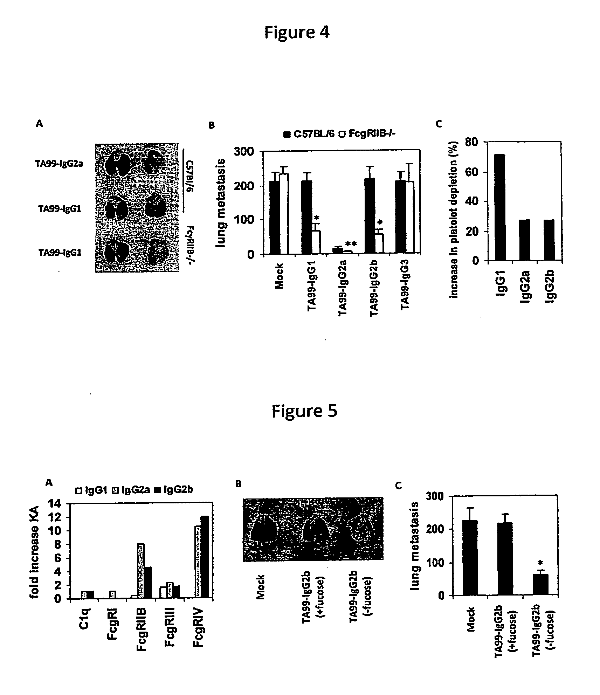

Fig. 4 illustrates differential isotype specific negative regulation by the inhibitory receptor FcγRIIB (A and B). C57BL/6 wild-type or Fcγ-receptor IIB deficient (FcγRIIB-/-) mice were injected with B16-F10 melanoma cells and treated with TA99-IgGl or IgG2a isotype switch variants (100 µg per injection). Lungs were prepared on day 15 after tumor cell injection. The experiment was done twice with 5 mice per group; representative lungs are shown in (A) and the quantification in (B). Asterisk indicates P<0.0001; double asterisk indicates P<0.05. (C) C57BL/6 or FcγRIIB-/- mice were injected with 2µg of the indicated 6A6-antibody isotype variants and platelet counts were determined before and 4 hours after antibody injection. Shown is the increase in platelet depletion for the different antibody isotypes in FcγRIIB-/- mice compared to wildtype animals. Shown is one representative out of three experiments with 5 mice per group.
Fig. 5 illustrates that the enhancement of the (A/I) ratio of modified antibodies increases their efficacy. (A) Shown is the fold increase in association constants (K_{A}) for the complement component Clq and FcγR-receptors I-IV in binding to fucose-containing TA99-IgGl, -IgG2a and -IgG2b isotypes compared to fucose-deficient TA99 isotype switch variants. (B and C) C57BL/6 mice were injected with B16-F10 melanoma cells and treated with TA99-IgG2b containing fucose or TA99-IgG2b deficient in fucose; Shields, R.L., et al., J Biol Chem 277, 26733-40 (2002) ; Shinkawa, T., et al., J Biol Chem 278, 3466-73. (2003); and Niwa R., et al., Cancer Res 64, 2127-33. (2004)) (50µg per injection). Lungs were prepared at day 15 after tumor cell injection. One representative lung out of 4 animals per group (B) and the quantification of the lung surface metastasis count is shown (C). Asterisk indicates P<0.0001.

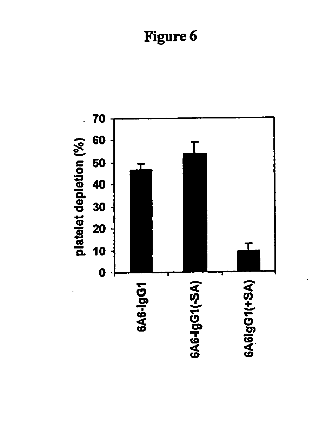

Figure 6 illustrates the effect of reducing sialic acid content on the in vivo cytotoxicity of an antibody. The effect of sialic acid residues in Asn-297 linked sugar side chains on antibody dependent cytotoxicity *in vivo* is described. Mice (n=4) were injected intravenously with 4 µg of the respective 6A6-IgG1 antibodies and platelet counts were determined before and 4 hours after antibody injection. Shown is the platelet depletion in percent 4 hours after injection of the antibody variants. Abbreviations: SA, sialic acid.

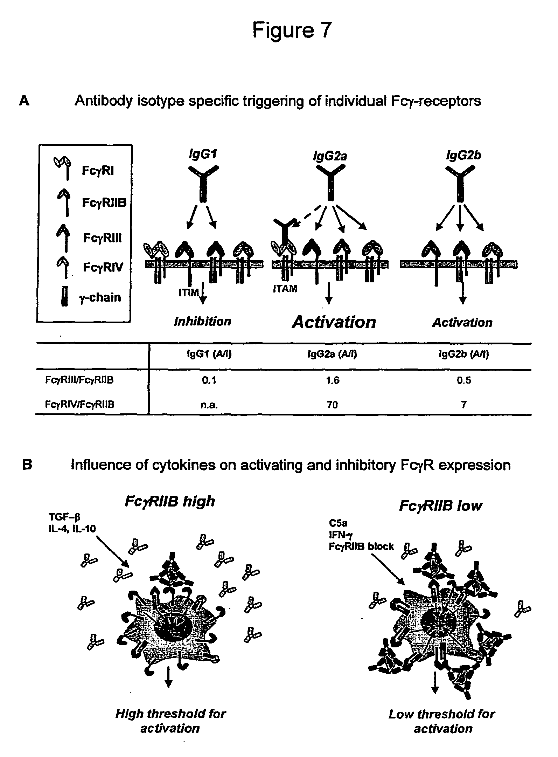

Fig. 7 illustrates factors that influence Fc-receptor dependent activities of antibody isotypes. Fig. 7A indicates individual antibody isotypes have different affinities for activating and inhibitory Fc receptors (see text). Red arrows indicate preferential interactions of the indicated antibody isotypes with cellular Fc-receptors; black arrows indicate lower affinity interactions. In the case of IgG2a the broken red arrow indicates that the interaction might be blocked as FcγRI is continuously occupied with monomeric IgG2a. The table summarizes the actual A/I ratios based on the affinities of the individual Fc-receptors for the respective antibody isotypes (Nimmerjahn et al., 2005). Fig. 7B indicates the ratio of activating to inhibitory Fc-receptors on immune cells such as DCs, macrophages and neutrophils is regulated by exogenous factors. Cytokines like IL-4, IL- 10 or TGF-f3 upregulate FcγRIIB thereby setting high thresholds for cell activation, whereas inflammatory mediators downregulate the inhibitory (shown in red) and upregulate the activating Fc-receptors (shown in green). For therapeutic approaches FcγRIIB mediated inhibition might be circumvented by using FcγRIIB-blocking antibodies.

### DESCRIPTION OF THE INVENTION

The present invention provides an advantageous strategy for enhancing effector function of therapeutic antibodies, particular anti-tumor, anti-viral, and anti-microbial (bacteria and unicellular parasites) antibodies, in mammals including humans. Disclosed are targets, methods, and reagents for specific activation and inhibition of Fc receptors in mammals including humans. Immunoglobulin G subclasses display significant differences *in vivo* in their ability to mediate effector responses, contributing to the variable activity of anti-microbial and anti-tumor antibodies and the pathogenic heterogeneity of autoantibodies. This differential activity results from the affinities of IgG subclasses for specific activating IgG Fc receptors as compared to their affinities for the inhibitory IgG Fc receptor. Applicants' invention is based on the discovery that in the human system, the cytotoxic activity of an antibody or variant thereof can be predicted by: determining a binding affinity of the antibody or variant thereof to an Fc activating receptor or receptors; determining a binding affinity of the antibody or variant thereof to a Fc inhibitory receptor, and calculating the ratio of said activating binding affinity to said inhibitory binding affinity (A/I ratio), wherein the magnitude of said ratio is an indication and predictive of the cytotoxic activity in vivo of the antibody or variant thereof. Activating receptors for the purposes of this calculation are selected from the group including FcRIIA, IIC or IIIA and the allelic variants of these receptors that are known to modify IgG binding. The high affinity FcRI is not included in these calculations. Thus, Applicants' invention provides a method of selecting a modified antibody for its cytotoxicity based on determining its ratio of binding affinity to activating Fc receptors versus inhibitory Fc receptors and then selecting the modified antibody with the highest ratio of activating to inhibitory receptor affinity.

These differential affinities result in antibodies with significantly different ratios of activation to inhibitory receptor binding that are predictive of the *in vivo* activity of a modified antibody and are important considerations in the design of therapeutic antibodies and vaccines.

A person who practices the method of the instant invention should keep in mind that the activating Fcγ receptor subtype should, preferably, be considered in determining the appropriate A/I ratio and, thus, the selection of the appropriate antibody. It is preferred that the A/I ratio should be calculated using the binding data for a receptor through which the antibody isotype exerts its effect. By way of example only and without any limitation, a person of the ordinary skill in the art will appreciate that if the FcγRIIIA/FcγRIIB ratio is higher than the FcγRIIA/FcγRIIB ratio this antibody isotype will exert its effect via the FcγRIIIA receptor and not the FcγRIIA receptor. Accordingly, the binding data for FcγRIIIA would be used for determining the appropriate A/I ratio.

To aid in the understanding of the invention, the following non-limiting definitions are provided:

### DEFINITIONS

Throughout the present specification and claims, the numbering of the residues in an immunoglobulin heavy chain is that of the EU index as in Kabat et al., Sequences of Proteins of Immunological Interest, 5th Ed. Public Health Service, National Institutes of Health, Bethesda, Md. (1991), which is expressly incorporated herein by reference. The "EU index as in Kabat" refers to the residue numbering of the human IgG1 EU antibody.

The term "native" or "parent" refers to an antibody comprising an amino acid sequence which lacks one or more of the Fc region modifications disclosed herein and which differs in effector function compared to a modified antibody as herein disclosed. The parent polypeptide may comprise a native sequence Fc region or an Fc region with pre-existing amino acid sequence modifications (such as additions, deletions and/or substitutions).

The term "Fc region" is used to define a C-terminal region of an immunoglobulin heavy chain. The "Fc region" may be a native sequence Fc region or a variant Fc region. Although the boundaries of the Fc region of an immunoglobulin heavy chain might vary, the human IgG heavy chain Fc region is usually defined to stretch from an amino acid residue at position Cys226, or from Pro230, to the carboxyl-terminus thereof.

The "CH2 domain" of a human IgG Fc region (also referred to as "Cγ2" domain) usually extends from about amino acid 231 to about amino acid 340. The CH2 domain is unique in that it is not closely paired with another domain. Rather, two N-linked branched carbohydrate chains are interposed between the two CH2 domains of an intact native IgG molecule. It has been speculated that the carbohydrate may provide a substitute for the domain-domain pairing and help stabilize the CH2 domain (Burton, Mol Immunol, 22, 161-206 (1985), which is incorporated herein by reference).

The "CH3 domain" comprises the stretch of residues C-terminal to a CH2 domain in an Fc region (i.e., from about amino acid residue 341 to about amino acid residue 447 of an IgG).

The term "hinge region" is generally defined as stretching from Glu216 to Pro230 of human IgG1 (Burton (1985). Hinge regions of other IgG isotypes may be aligned with the IgG1 sequence by placing the first and last cysteine residues forming inter-heavy chain S--S bonds in the same positions.

The "lower hinge region" of an Fc region is normally defined as the stretch of residues immediately C-terminal to the hinge region, i.e., residues 233 to 239 of the Fc region. Prior to the present invention, FcγR binding was generally attributed to amino acid residues in the lower hinge region of an IgG Fc region.

The term "binding domain" refers to the region of a polypeptide that binds to another molecule. In the case of an FcR, the binding domain can comprise a portion of a polypeptide chain thereof (e.g., the α chain thereof) which is responsible for binding an Fc region. One useful binding domain is the extracellular domain of an FcR chain.

A "functional Fc region" possesses an "effector function" of a native sequence Fc region. Exemplary "effector functions" include Clq binding; complement dependent cytotoxicity; Fc receptors binding; antibody-dependent cell-mediated cytotoxicity (ADCC); phagocytosis; down regulation of cell surface receptors (e.g., B cell receptor; BCR), etc. Such effector functions generally require the Fc region to be combined with a binding domain (e.g., an antibody variable domain) and can be assessed using various assays as herein disclosed, for example.

A "native sequence Fc region" comprises an amino acid sequence identical to the amino acid sequence of an Fc region found in nature. A "variant Fc region" comprises an amino acid sequence which differs from that of a native sequence Fc region by virtue of at least one "amino acid modification" as herein defined. Preferably, the variant Fc region has at least one amino acid substitution compared to a native sequence Fc region or to the Fc region of a parent polypeptide, e.g., from about one to about ten amino acid substitutions, and preferably from about one to about five amino acid substitutions in a native sequence Fc region or in the Fc region of the parent polypeptide. The variant Fc region herein will preferably possess at least about 80% homology with a native sequence Fc region and/or with an Fc region of a parent polypeptide, and most preferably at least about 90% homology therewith, more preferably at least about 95% homology therewith

The term "altered glycosylation" refers to an antibody, as defined above, be it native or modified, in which the carbohydrate addition to the heavy chain constant region is manipulated to either increase or decrease specific sugar components. For example, antibodies prepared in specific cell lines may be deficient in the attachment of sugar moieties such as fucose and sialic acid. Alternatively, antibodies can be isolated on specific lectin affinity reagents to enrich or deplete for specific sugar moieties. For example, the lectin isolated from Sambuccus nigra will bind sialic acid and permit the enrichment or depletion of antibodies with this specific sugar residue attached. Enzymatic treatment of antibodies may also enrich or deplete specific sugar residues such as neuraminidase treatment to remove sialic acid or sialytransferase to introduce sialic acid residues.

The term "Fc region-containing polypeptide," refers to a polypeptide, such as an antibody or immunoadhesin (see definitions below), which comprises an Fc region.

The terms "Fc receptor" or "FcR" are used to describe a receptor that binds to the Fc region of an antibody. The preferred FcR is a native sequence human FcR. Moreover, a preferred human FcR is one which binds an IgG antibody (a gamma receptor) and includes receptors of the human FcγRI, FcγRII, and FcγRIII subclasses, including allelic variants and alternatively spliced forms of these receptors. FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain (see review in Daron, Annu Rev Immunol, 15, 203-234 (1997); FcRs are reviewed in Ravetch and Kinet, Annu Rev Immunol, 9, 457-92 (1991); Capel et al., Immunomethods, 4, 25-34 (1994); and de Haas et al., J Lab Clin Med, 126, 330-41 (1995), each of which is incorporated herein by reference)..

"Antibody-dependent cell-mediated cytotoxicity" and "ADCC" refer to an in vitro or in vivo cell-mediated reaction in which nonspecific cytotoxic cells that express FcRs (e.g., monocytic cells such as natural killer (NK) cells and macrophages) recognize bound antibody on a target cell and subsequently cause lysis of the target cell. In principle, any effector cell with an activating FcγR can be triggered to mediate ADCC. One such cell the NK cell, express FcγRIII only, whereas monocytes, depending on their state of activation, localization, or differentiation, can express FcγRI, FcγRII, and FcγRIII. FcR expression on hematopoietic cells is summarized in Ravetch and Bolland, Annu Rev Immunol, (2001), which is incorporated herein by reference.

"Human effector cells" are leukocytes which express one or more FcRs and perform effector functions. Preferably, the cells express at least FcγRIII and perform ADCC effector function. Examples of human leukocytes which mediate ADCC include peripheral blood mononuclear cells (PBMC), natural killer (NK) cells, monocytes, and neutrophils, with PBMCs and NK cells being preferred. The effector cells may be isolated from a native source thereof, e.g., from blood or PBMCs as described herein.

The term "antibody" is used in the broadest sense and specifically covers monoclonal antibodies (including full length monoclonal antibodies), polyclonal antibodies, multispecific antibodies (e.g., bispecific antibodies), and antibody fragments so long as they exhibit the desired biological activity.

"Antibody fragments", as defined for the purpose of the present invention, comprise a portion of an intact antibody, generally including the antigen binding or variable region of the intact antibody or the Fc region of an antibody which retains FcR binding capability. Examples of antibody fragments include linear antibodies; single-chain antibody molecules; and multispecific antibodies formed from antibody fragments. The antibody fragments preferably retain at least part of the hinge and optionally the CH1 region of an IgG heavy chain. More preferably, the antibody fragments retain the entire constant region of an IgG heavy chain, and include an IgG light chain.

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations that typically include different antibodies directed against different determinants (epitopes), each monoclonal antibody is directed against a single determinant on the antigen. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler and Milstein, Nature, 256, 495-497 (1975), which is incorporated herein by reference, or may be made by recombinant DNA methods (see, e.g., U.S. Patent No. 4,816,567, which is incorporated herein by reference). The monoclonal antibodies may also be isolated from phage antibody libraries using the techniques described in Clackson et al., Nature, 352, 624-628 (1991) and Marks et al., J Mol Biol, 222, 581-597 (1991), for example, each of which is incorporated herein by reference.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (see U.S. Patent No. 4,816,567; Morrison et al., Proc Natl Acad Sci USA, 81, 6851-6855 (1984); Neuberger et al., Nature, 312, 604-608 (1984); Takeda et al., Nature, 314, 452-454 (1985); International Patent Application No. PCT/GB85/00392, each of which is incorporated herein by reference).

"Humanized" forms of non-human (e.g., murine) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, Fv framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FR residues are those of a human immunoglobulin sequence. The humanized antibody optionally also will comprise at least a portion of an immunoglobulin constant region (Fc), typically that of a human immunoglobulin. For further details, see Jones et al., Nature, 321, 522-525 (1986); Riechmann et al., Nature, 332, 323-329 (1988); Presta, Curr Op Struct Biol, 2, 593-596 (1992); U.S. Patent No. 5,225,539, each of which is incorporated herein by reference.

### Generation of Modified Antibodies

Modified antibodies include those in which specific amino acid substitutions, additions or deletions are introduced into a parental sequence through the use of recombinant DNA techniques to modify the genes encoding the heavy chain constant region. The introduction of these modifications follows well-established techniques of molecular biology, as described in manuals such as Molecular Cloning (Sambrook and Russel, (2001)). In addition, modified antibodies will include those antibodies which have been selected to contain specific carbohydrate modifications, obtained either by expression in cell lines known for their glycosylation specificity (Stanley P., et al., Glycobiology, 6, 695-9 (1996); Weikert S., et al., Nature Biotechnology, 17, 1116-1121 (1999); Andresen DC and Krummen L., Current Opinion in Biotechnology, 13, 117-123 (2002)) or by enrichment or depletion on specific lectins or by enzymatic treatment (Hirabayashi et al., J Chromatogr B Analyt Technol Biomed Life Sci, 771, 67-87 (2002); Robertson and Kennedy, Bioseparation, 6, 1-15 (1996)). It is known in the art that quality and extent of antibody glycosylation will differ depending on the cell type and culture condition employed. (For example, Patel et al., Biochem J, 285, 839-845 (1992)) have reported that the content of sialic acid in antibody linked sugar side chains differs significantly if antibodies were-produced as ascites or in serum-free or serum containing culture media. Moreover, Kunkel et al., Biotechnol Prog, 16, 462-470 (2000) have shown that the use of different bioreactors for cell growth and the amount of dissolved oxygen in the medium influenced the amount of galactose and sialic acid in antibody linked sugar moieties. These studies, however, did not address how varying levels of sialic acid residues influence antibody activity in vivo. Creation of desialylated antibodies.

Most of the carbohydrates on antibodies are N-linked at Asn297 in the CH2 domain. High percentages of the Fc-associated carbohydrates in humans, mice and from hybridomas are incompletely processed, varying in structure-type (complex- or high mannose-type), in the amounts of sialic acid, galactose and/or GlcNAc residues in the outer branches, and in core fucosylation. Only 12-15% of Fc-associated carbohydrates are sialylated. The level of antibody glycosylation and specifically galactosylation and sialylation might also vary significantly in human autoimmune disease. For example IgG antibodies in human rheumatoid arthritis have been found to contain decreased levels of galactose and sialic acid in antibody linked sugar moieties (Parekh RB, et al., Nature, 316, 452-457 (1985); Tsuchiya et al., J Immunol., 151, 1137-1146 (1993); Matsumoto et al., J Biochem (Tokyo), 128, 621-628 (2000); Rademacher et al., Proc Natl Acad Sci USA, 91, 6123-6127 (1994)) The antibodies of the present invention can be further purified or modified so that they have a decreased amount of sialic acid compared to unmodified and/or unpurified antibodies. Multiple methods exist to reach this objective. In one method, the source of the recombinantly expressed, antigen-specific antibody is passed through an affinity chromatography column containing a lectin, known to bind sialic acid. Lectin affinity chromatography is widely known in the art and is described in, for example, Schmauser at al., Glycobiology, 12, 1295-305 (1999). As a result of this technique, the sialylated portion of the antibodies will be retained in the column while desialylated portion will pass through. A person of ordinary skill in the art will appreciate that the affinity chromatography method described above can be used not only with recombinantly expressed antigen-specific antibodies, but with unspecific unpurified sources as well, such as, for example, intravenous immunoglobulin ("IVIG") preparations. We have observed that approximately 15-20% of IVIG is sialylated. Accordingly, this invention provides for antibodies with lower sialic acid content produced from IVIG and the method of production of such antibodies.

Further, one may employ an enzymatic reaction with sialidase, such as, for example, *Arthrobacter ureafacens* sialidase. The conditions of the reaction are generally described in the U.S. Patent No. 5,831,077. Other non-limiting examples of suitable enzymes are neuraminidase and N-Glycosidase F, as described in Schloemer et al., J. Virology, 15(4), 882-893 (1975) and in Leibiger et al., Biochem J., 338, 529-538 (1999), respectively. The desialylated antibodies may be further purified by using affinity chromatography, as described above.

Still further, if the starting material for the antibody samples comprises antibodies grown in cell culture, desialylation can be achieved by modifying culture media conditions. For example, decreasing the sialic acid content of the mature glycoprotein produced by mammalian cell culture can be achieved by increasing cell specific productivity of the cell culture. The cell specific productivity is increased by providing a cell culture which either contains about 6 mM to about 12 mM of an alkanoic acid or salt thereof or has the osmolality at about 450-600 mOsm/kg. This method is described in, e.g., U.S. Patent No. 6,656,466.

Also, antibodies of interest may be expressed in cell lines having deficiency in sialylating these antibodies. Suitable examples of these cells are Lec 2 and Lec 3 mutants of CHO cells. (See for example, Stanley, Mol Cel Biol, 9(2), 377-383 (1989)). It has been shown that approximately 4% of glycoproteins, including antibodies grown in these cells are sialylated (Jassal et al., Biochemical and Biophysical Research Communications, 286, 243-249 (2001); Lund et al., J Immunol, 157(11), 4963-4969 (1996)).

A person of ordinary skill in the art will appreciate that different combinations of desialylation methods, disclosed above, can lead to production of antibodies with extremely low level of sialylation. For example, one can express antibodies in sialylation-deficient cell lines, such as Lec 2 and Lec 3, and then further enrich the desialylated fraction of these antibodies by, for example, desialylating the antibodies in an enzymatic reaction followed by affinity chromatography using lectin-containing columns. Similarly, an enzymatic reaction followed by affinity chromatography may be used for IVIG source of antibodies.

To examine the extent of glycosylation on these modified antibodies, the antibodies can be purified and analyzed in SDS-PAGE under reducing conditions. The heavy chains of the modified antibodies will migrate at faster rates, due to desialylation, compared to that of the parent antibody. From the relative migration rates of the peptides in SDS-PAGE, which are inversely proportional to the molecular sizes, the extent of glycosylation at the different sites can be estimated.

### Assays

The A/I ratio of the modified antibody candidates of the present invention can be readily determined by any number of assays widely known in the art, such as for example, a competition or sandwich ELISA, a radioimmunoassay, a dot blot assay, a fluorescence polarization assay, a scintillation proximity assay, a homogeneous time resolved fluorescence assay, a resonant mirror biosensor analysis, and a surface plasmon resonance analysis.

Generally, in one embodiment, the modified antibody candidates, the activating Fc receptors, and/or inhibitory Fc receptors is directly labeled with a detectable label and may be detected directly. In another embodiment, neither the modified antibody candidates nor the activating Fc receptor nor inhibitory Fc receptor is labeled. Instead, a secondary antibody or other molecule that can bind the modified antibody candidates or one of the activating Fc receptor or the inhibitory Fc receptor is labeled. As is well known to one of skill in the art, a secondary antibody is chosen that is able to specifically bind the specific species and class of the modified antibody candidates or the activating or inhibitory Fc receptor. For example, if the modified antibody candidates are human IgGs, then the secondary antibody may be an anti-human-IgG. The amount of an antibody-receptor complex in the biological sample can be detected by detecting the presence of the labeled secondary antibody. Other molecules that can bind to antibodies include, without limitation, Protein A and Protein G, both of which are available commercially, for example, from Pierce Chemical Co. (Rockford, IL.)

Suitable labels for the modified antibody candidates, the activating Fc receptor, the inhibitory Fc receptor or secondary antibody are widely known in the art and include various enzymes, prosthetic groups, fluorescent materials, luminescent materials, magnetic agents and radioactive materials. Examples of suitable enzymes include horseradish peroxidase, alkaline phosphatase, p-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; examples of a luminescent material include luminol luciferin, pyrogallol, or isoluminol; an example of a magnetic agent includes gadolinium; and examples of suitable radioactive material include ¹²⁵I, ¹³¹I, ³⁵S or ³H.

### Competition ELISA.

Binding of modified antibody candidates to Fc activating and Fc inhibitory receptors can be measured by a competition ELISA. In this method, it would be advantageous to use an antibody with known A/I ratios as a control substrate for reaction with either the activating Fc receptors or the inhibitory Fc receptors, which are labeled, and use the modified antibody candidates as competitors. In another embodiment, the activating and the inhibitory Fc receptors would be unlabeled and a labeled secondary antibody against either the activating Fc receptor or the inhibitory Fc receptor may be added to the reaction in the second step.

Alternatively, one may use the modified antibody candidates as a substrate and use both the activating Fc receptors and the inhibitory Fc receptors as competitors. The activating receptor and the inhibitory Fc receptors may be labeled as discussed above. In yet another embodiment of this method, a labeled secondary antibody against either the activating Fc receptor or the inhibitory Fc receptor may be added to the reaction in the second step.

### Sandwich ELISA.

In a Sandwich ELISA, the activating or the inhibitory Fc receptor is immobilized on a solid carrier and is brought into contact with a liquid containing the modified antibody candidates. Then the quantity of the bound modified antibody candidates is determined by adding a second antibody which is labeled with a detectable label such as a radioactive atom, a fluorescent or luminescent group or, in particular, an enzyme (for example horseradish peroxidase (HRP)). If the modified antibody candidates are human IgGs, then the second antibody may be an anti-human-IgG antibody. The amount of the bound second antibody is then determined by measuring the activity, for example the enzyme activity of the label. This activity is a measure of binding of the modified antibody candidates to the activating Fc receptor or the inhibitory Fc receptor.

Alternatively, the modified antibody candidates may be immobilized and a mixture containing the activating Fc receptor or the inhibitory Fc receptor added to the modified antibody candidates. In this embodiment, the secondary antibody would be used against the activating Fc receptor or Fc receptor.

It is important that the secondary antibody binds an epitope of its target, which is not affected by binding of the modified antibody candidates to the activating or the inhibitory Fc receptor.

### Radioimmunoassay.

A radioimmunoassay can also be used in determining binding affinities of the modified antibody candidates to activating Fc receptors and inhibitory Fc receptors. In the first step of this method, radioactively-labeled modified antibody candidates are mixed with the activating or the inhibitory Fc receptors. The antibodies may be labeled by, for example, radioactive iodine attached to tyrosine moieties. In the second step, non-labeled modified antibody candidates are added to the mix in the known quantities and antigen-antibody complexes are removed from the mixture by, for example, precipitation. The amount of labeled unbound modified antibody candidates is then determined.

### Dot Blot analysis.

A dot blot procedure can also be used for this analysis. The use of the dot blot procedure eliminates the need to perform electrophoresis and allows rapid analysis of a large number of samples. In one embodiment of this method, different dilutions of the activating Fc receptors or the inhibitory Fc receptors can be placed on a membrane, such as, for example, nitrocellulose membrane, and contacted with radioactively or fluorescence labeled modified antibody candidates.

A person skilled in the art will appreciate that the modified antibody candidates do not have to be labeled. In that case, after incubating the membrane-bound activating or inhibitory Fc receptor with the modified antibody candidates, a secondary antibody, which is labeled, is added to the reaction. The amount of signal produced by the label (radioactivity, light, color, etc) can then be quantified.

### Fluorescence polarization assay.

This assay is based on the principle that a fluorescent tracer, when excited by plane polarized light of a characteristic wavelength, will emit light at another characteristic wavelength (i.e., fluorescence) that retains a degree of the polarization relative to the incident stimulating light that is inversely related to the rate of rotation of the tracer in a given medium. As a consequence of this property, a tracer substance with constrained rotation, such as in a viscous solution phase or when bound to another solution component, such as an antibody with a relatively lower rate of rotation, will retain a relatively greater degree of polarization of emitted light than if in free solution. Thus, a person of skill in the art can label the activating Fc receptor or the inhibitory Fc receptor with an appropriate label and contact the labeled receptor with the modified antibody candidates. The fluorescence polarization assays can be conducted in commercially available automated instruments such as IMx^{®}, TDx^{®}, and TDxFLx^{™}. (Abbott Laboratories, Abbott Park, IL).

### Scintillation proximity assay.

The activating Fc receptor or the inhibitory Fc receptor can be coupled to a scintillation-filled bead. Binding of radio-labeled modified antibody candidates to the activating Fc receptors or the inhibitory Fc receptors would result in emitted light which can be quantified on a scintillation counter. Commercial kits for the scintillation proximity assay are currently available and may be purchased from, for example, Amersham Life Science (Piscataway, NJ).

### Homogeneous specific binding assay.

In this assay, a conjugate is formed between a binding substance (i.e. the modified antibody candidates or the activating or inhibitory Fc receptor) and coupled to a label, which is chosen in such a way that it behaves differently depending on whether the binding substance is bound or free. Thus, in one embodiment of the method, different samples containing known amounts of labeled activated Fc receptors or labeled inhibitory Fc receptor in a liquid medium can be contacted with a solid matrix coated with or impregnated with the modified antibody candidates. In another embodiment, the activating Fc receptor or the inhibitory Fc receptor can be placed onto a solid carrier and contacted with different liquid samples containing known amounts of the modified antibody candidates which are labeled. Examples of labels suitable for this method are chemiluminescent compounds and enzymes, as disclosed above. Change in chemiluminescence can be measured, thus reflecting on the relative amount of bound modified antibody candidates.

### Surface plasmon resonance analysis.

This method is based on quantifying the intensity of electromagnetic waves, also called surface plasmon waves, which may exist at the boundary between a metal and a dielectric. Such waves can be exited by light which has its electric field polarized parallel to the incident plane (i.e., transverse magnetic (TM) polarized).

In this method, one of the reagents (i.e., the modified antibody candidates or the activating Fc receptor or the inhibitory Fc receptor) is coupled to the dextran layer (covering the metal film) of a sensor chip and solutions containing different concentrations of the other reagent (i.e. the activating Fc receptor or the inhibitory receptor or the antibody, respectively) are allowed to flow across the chip. Binding (association and dissociation) is monitored with mass sensitive detection. BIACORE^{®} (Biacore AB, Uppsala, Sweden) equipment can be used for this method. The application of this method to the present invention is described in detail in Example 1 of the disclosure.

Other modifications of these assays, not disclosed in this application will be apparent to a person of ordinary skill in the art. The claims of the present invention include all such modifications.

### Therapeutic Formulations

Therapeutic formulations of the modified antibody can be prepared for storage by mixing the modified antibody having the desired degree of purity with optional physiologically acceptable carriers, excipients or stabilizers (see, e.g., Remington's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenyl, butyl or benzyl alcohol; alkyl parabens such as methyl or propyl paraben; catechol; resorcinol; cyclohexanol; 3-pentanol; and m-cresol); low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN^{™}, PLURONICS^{™} or polyethylene glycol (PEG).

The formulations herein may also contain more than one active compound as necessary for the particular indication being treated, preferably those with complementary activities that do not adversely affect each other. Such molecules are suitably present in combination in amounts that are effective for the purpose intended.

The active ingredients may also be entrapped in a microcapsule prepared, for example, by coacervation techniques or by interfacial polymerization, for example, hydroxymethylcellulose or gelatin-microcapsule and poly-(methylmethacylate) microcapsule, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

The formulations to be used for in vivo administration must be sterile. This is readily accomplished by filtration through sterile filtration membranes.

Sustained-release preparations may also be prepared. Suitable examples of sustained-release preparations include semipermeable matrices of solid hydrophobic polymers containing the modified antibody, which matrices are in the form of shaped articles, e.g., films, or microcapsule. Examples of sustained-release matrices include polyesters, hydrogels (for example, poly(2-hydroxyethyl-methacrylate), or poly(vinylalcohol)), polylactides (see, e.g., U.S. Pat. No. 3,773,919), copolymers of L-glutamic acid and y ethyl-L-glutamate, non-degradable ethylene-vinyl acetate, degradable lactic acid-glycolic acid copolymers such as the LUPRON DEPOT^{™} (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S--S bond formation through thio-disulfide interchange, stabilization may be achieved by modifying sulfhydryl residues, lyophilizing from acidic solutions, controlling moisture content, using appropriate additives, and developing specific polymer matrix compositions.

### EXAMPLES

Specific embodiments of the present invention will now be described. The examples are illustrative only, and are not intended to limit the remainder of the disclosure in any way.

### Example 1

### In Vivo Activity Of IgG Subclasses Dependant On FcγR Specificity

To address the role of individual FcγRs to the *in vivo* activities of specific IgG subclasses a series of antibodies were constructed for two defined epitopes, in which the V_{H} regions of the cloned hybridoma recognizing either the melanosome gp75 antigen (TA99 family) or anti-platelet integrin antigen (6A6 family) were grafted onto the C57BL/6-derived G1, 2a, 2b or 3 constant regions and co-expressed with the appropriate light chains in 293 T cells (Nimmerjahn et al., Immunity 23, 41-51 (2005) ; Vijayasaradhi et al. , J. Exp Med 171, 1375-80 (1990); and Clynes et al., Proc Natl Acad Sci USA 95, 652-6 (1998)). These recombinant antibodies were purified and tested for binding affinity to their cognate antigen (Table 1) and to soluble, recombinantly expressed FcγR I, II, III or IV by surface plasmon resonance or to transfected cells expressing a heterologous Fc receptor. Switching IgG constant regions did not affect the binding affinity of the resultant antibodies to their respective antigens (Table 1).

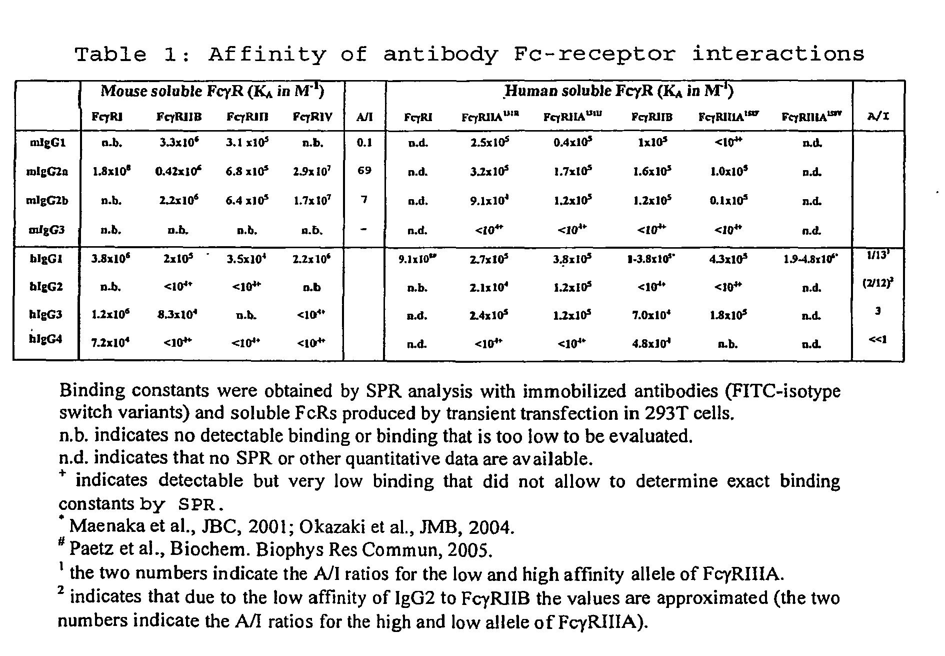

In contrast, specific differences in binding affinity of each subclass to specific FcγRs were observed. For example, IgG1 bound with 10-fold higher affinity to the inhibitory receptor FcγRIIB than to its activation counterpart, FcγRIII, while IgG2a and 2b displayed the reverse pattern, binding with 10-fold higher affinity for the activation receptor FcγRIV than to the inhibitory receptor FcγRIIB. IgG3 did not bind to any of the known FcγRs. The ratio of activation to inhibitory binding (A/I), as shown in Table 2, thus can differ by as much as 2 orders of magnitude between IgG subclasses and FcRs.

Similarly to mouse antibodies, human antibodies also displayed specific differences in binding affinity of each subclass to specific FcγRs were observed. For example, IgG4 bound with at least 4.8-fold higher affinity to the inhibitory receptor FcγRIIB than to its activation counterparts, FcγRIIIA and FcγRIIA, while IgG2 displayed the reverse pattern, binding with 12-fold higher affinity for the activation receptor FcγRIIA than to the inhibitory receptor FcγRIIB. IgG3 showed approximately 1.5-fold higher affinity to activating receptors, FcγRIIA and FcγRIIIA than to the inhibitory receptor FcγRIIB. IgG1 also shown approximately 1.5-2 fold higher affinity to activating receptors, FcγRIIA and FcγRIIIA than to the inhibitory receptor FcγRIIB.

**Table 2: Affinities of soluble human Fc-receptors to human Fc-switch variants of the 4-4-20 antibody lacking fucose**

| | hFcγRIIB | hFcγRIIA(131H) | hFcγRIIIA(158F) |
|---|---|---|---|
| | K_{A}/K_{D} | K_{A}/K_{D} | K_{A}/K_{D} |
| FITC-IgG1-Fucose | 7.81x10⁴/1.28x10⁻⁵ | 2.39x10⁵/4.19x10⁻⁶ | 5.97x10⁶/1.68x10⁻⁷ |
| FITC-IgG2-Fucose | 2.54x10⁴/3.94x10⁻⁵ | 1.35x10⁵/7.4x10⁻⁶ | 1.44x10⁵/6.93x10⁻⁶ |

Materials and Methods:
Mice: C57BL/6 and C57BL/6-129SF2/J mice were obtained from the Jackson Laboratory (Bar Harbor, ME). γ^{-/-}, FcγRIIB^{-/-}
   and FcγRIII^{-/-} mice were generated in our laboratory and backcrossed for 12 generations to the C57BL/6 background. FcγRI^{-/-} 129/B6 mice were generously provided by Dr. Hogarth (The Austin Research Institute, Victoria, Australia). FcγRI/III^{-/-} mice were generated in our laboratory by crossing FcγRI^{-/-} with FcγRIII^{-/-} mice and subsequent selection for double knockout animals. CR2-/-, C3-/- and C4-/- knockout mice were provided by Michael Carroll (CBR Institute for Biomedical Research, Harvard Medical School). Female mice at 2 to 4 months of age were used for all experiments and maintained at the Rockefeller University animal facility. All experiments were done in compliance with federal laws and institutional guidelines and have been approved by the Rockefeller University (New York, New York).

Cell culture: 293T, CHO-K1, B16-F10 and YB2/0 cells were cultured according to ATCC guidelines.

Antibodies and recombinant proteins: The 6A6 and TA99 antibody isotype switch variants and soluble Fcγ-receptors and gp75 were produced by transient transfection of 293T cells and subsequent purification from culture supernatants as described (Nimmerjahn, F., et al., (2005)). For generation of TA99 antibody variants lacking fucose YB2/0 cells were stably transfected with the respective TA99 heavy and light chains. Fucose content of antibodies was verified by immunoblotting with biotinylated aleuria aurantia lectin (Vector laboratories) followed by detection with streptavidin-AP (Roche). Antibodies enriched or depleted for sialic acid residues were generated by affinity chromatography with sambucus nigra lectin (Vector laboratories). Sialic acid content was verified by immunoblotting with biotinylated sambucus nigra lectin (Vector laboratories). Purified Clq was from Calbiochem.

The FcγRIV-blocking antibody 9E9 has been described before *Id.* A hamster IgGl anti-TNP antibody was used as an isotype control antibody (Pharmingen).

Surface plasmon resonance (SPR) analysis: A Biacore 3000 biosensor system was used to assay the interaction of soluble mouse Fcγ-receptors I, II, III and IV and soluble gp75 with the indicated antibody isotypes. Additionally, soluble human Fcγ-receptors IIA (131H-allele), IIB and IIIA (158F-allele) were used to measure the affinity to human IgG antibody isotypes. Antibodies or BSA as a control protein were immobilized at high and low densities to flow cells of CM5 sensor chips (Biacore) by standard amine coupling as suggested by the manufacturer. Soluble Fcγ-receptors were injected at 5 different concentrations through flow cells at room temperature in HBS-EP running buffer (10 mM Hepes, pH 7.4, 150 mM NaCl, 3.4 mM EDTA, and 0.005% surfactant P20) at a flow rate of 30 µl/min. Soluble Fc-receptors were injected for 3 minutes and dissociation of bound molecules was observed for 10 minutes. Background binding to control flow cells was subtracted automatically. Control experiments were performed to exclude mass transport limitations. Affinity constants were derived from sensorgram data using simultaneous fitting to the association and dissociation phases and global fitting to all curves in the set. As described for soluble human Fc-receptors a 1:1 Langmuir binding model closely fitted the observed sensorgram data and was used in all experiments *Id.* Alternatively, soluble Fc-receptors were immobilized to sensor chips with the same result described above.

In vivo model systems: The platelet depletion model: Experiments were performed essentially as described before. *Id.* Briefly, mice were injected intravenously with 4 µg of the recombinant 6A6 antibody isotype switch-variants diluted in 200 µl of PBS. Alternatively, mice were injected with 2 µg of the 6A6 antibody variants to study FcγRIIB-mediated negative regulation of antibody functions *in vivo*. Platelet counts before injection and at indicated time points after injection were determined by blood collection (40 µl) from the retro-orbital plexus and measuring platelet counts of a 1:10 dilution in PBS/5%BSA in an Advia 120 haematology system (Bayer). To block FcγRIV *in vivo* mice were injected 30 minutes before administration of the 6A6 antibody variants with 200 µg of the blocking FcγRIV antibody 9E9 or with 200 µg of a hamster isotype control antibody (Pharmingen).

The B16-F10 lung metastasis model: Experiments were performed as described (Vijayasaradhi et al., J Exp Med 171, 1375-80. (1990); and Clynes et al., Proc Natl Acad Sci USA 95, 652-6. (1998)) with minor modifications. Mice were injected with 5x10⁵ B16-F10 tumor cells intravenously and either left untreated or were injected with the indicated amounts of isotype control (Sigma) or TA99-isotype switch variants on days 0, 2, 4, 7, 9, 11 intraperitoneally. To block FcγRIV in vivo mice were injected with 200 µg of the 9E9 or the respective hamster isotype control antibody on days 0, 2, and 4 intravenously. On day 15 after tumour cell injection mice were sacrificed and lungs were analyzed for the presence of surface metastasis by an investigator blinded for the experimental setup.

Statistical analysis: The paired Student's t-test was used for determining significance of the results.

### Example 2

### A/I Ratios Of Antibody Variants Predictive Of In Vivo

### Biological Activity

To determine how these differences in binding affinities relate to *in vivo* biological activity, the ability of these antibodies to mediate tumor clearance or platelet depletion was investigated. As seen in Figure 1, both TA99 (Fig. 1A and B) and 6A6 (Fig. 1C and D) with IgG2a constant regions display enhanced tumor or platelet clearance, respectively, as compared to these antibodies with IgG1 constant regions. IgG2a and 2b are equivalent in their ability to mediate platelet clearance, while IgG2a results in enhanced tumor ADCC in the metastatic melanoma model as compared to IgG2b. The hierarchy of activity for the IgG subclasses is thus IgG2a≥IgG2b>IgG1>>IgG3. The mechanism of this differential activity was determined by repeating these experiments in specific activating FcγR or complement deficient strains. No differences in *in vivo* activity were observed for IgG1, 2a or 2b in complement deficient strains (C4, C3 or CR1/2) (Fig. 2). In contrast, IgG1, 2a and 2b were all dependent on activating FcγR expression, since activity was abrogated in the common γ chain deficient background (Fig. 3A, B and E). While IgG2a activity could result from its ability to bind with high affinity to FcγRI, intermediate affinity to FcγRIV or low affinity to FcγR III, only FcγRIV binding was relevant to its *in vivo* activity (Fig. 3C, D and E). Similarly, IgG2b activity was FcγRIV dependents and FcγRIII independent (Fig. 3E). In contrast, IgG1 mediated effector activity was exclusively FcγRIII dependent (Fig. 3E).

The balance of activation to inhibitory receptor expression has been shown to determine the threshold for IgG mediated effector cell triggering (Ravetch and Lanier, Science 290, 84-9 (2000)). The binding affinities of IgG subclasses to the inhibitory receptor vary by a factor of 10, suggesting that a differential dependence of the subclasses on the inhibitory effect of FcγRIIB might be observed. As seen in Figure 4, IgG1 displays the greatest enhancement in activity in mice lacking the inhibitory receptor in both the tumor clearance and platelet depletion models (Fig. 4A-C), while IgG2a shows the smallest enhancement in both models. The magnitude of IgG2b enhancement in FcγRIIB deficient strains differs in the two models, showing significant enhancement in the tumor clearance model and minimal enhancement in the platelet depletion model. This difference is likely due to the intermediate A/I ratio of this receptor rendering it more sensitive to the levels of surface expression of FcγRIIB on the specific effector cells mediating the *in vivo* responses. Since different populations of effector cells are responsible for the biological responses in the two models, the IgG2b data support our previous observations that RIIB levels are minimal on splenic macrophages, the cell type responsible for platelet clearance (Nimmerjahn et al., Immunity 23, 41-51. (2005); and Samuelsson et al., Science 291, 484-6. (2001)), and higher on alveolar macrophages, the relevant effector cells in the metastatic melanoma model (Shushakova et al., J Clin Invest 110, 1823-30 (2002)). These results demonstrate the predictive value of the A/I ratio in determining the contribution of inhibitory signaling to *in vivo* activities. A high A/I ratio, as found for IgG2a, renders the antibody essentially insensitive to differences in FcγRIIB expression on different effector cell populations, while a low A/I ratio, as found for IgG1, maximizes the role of FcγRIIB. For antibodies with intermediate A/I ratios, like IgG2b, the *in vivo* activity, will be determined by the specific effector cell involved in the response, reflecting the differences in FcγRIIB levels and its regulation by the cytokine milieu. This difference in FcγRIIB dependence for IgG1 and IgG2 may reflect the biological roles of these subclasses *in vivo,* insuring that the most abundant subclass, IgG1, is under tight regulation by an inhibitory receptor, thus preventing effector cell activation in the absence of a second signal that down regulates FcγRIIB and lowers the threshold for activation. Such second signals are provided by pro- and antiinflammatory cytokines and chemokines which have been demonstrated to alter the levels of surface expression of activation or inhibitory receptors (Shushakova (2002)). In contrast, the role of inhibitory receptor expression on IgG2a potency, and to a lesser extent, IgG2b, is less significant, reflecting the effector bias of T_{H1} cytokines which induce both IgG2a switching and FcγRIV expression.

### Example 3

### Modified Antibody With A Lower Amount Of Fucose And A Greater A/I Ratio Compared To Unmodified Antibody

The relationship between the A/I ratio of IgG subclasses and *in vivo* activity was further tested using modified IgG constant regions. FcR binding to IgG is dependent on the presence of N-linked glycosylation at position 297; deglycosylation abrogates all FcR binding (Krapp, J Mol Biol 325, 979-89 (2003)). However, selective removal of specific carbohydrates, such as fucose, has been suggested to modify human IgG1 binding to human FcγRIII and thus to NK cell mediated ADCC *in vitro* (Shields, R.L., et al., J Biol Chem 277, 26733-40. (2002); T. Shinkawa et al., J Biol Chem 278, 3466-73 (2003); and Niwa et al., Cancer Res 64, 2127-33 (2004)). Fucose-deficient TA99-IgGl, 2a and 2b were prepared and their binding to FcγRI, II, III and IV was compared. Clq or antigen binding was not affected by the lack of fucose as described before (Shields (2002)). However, as shown in Figure 5 and Table 2, fucose deficient antibodies differed in their binding affinities to their cognate FcγRs, with TA99-IgG1 with or without fucose displaying minimal differences in binding to FcRIIB and III, while IgG2a and 2b fucose deficient antibodies bound with an order of magnitude higher affinity to FcRIIB and FcRIV as compared to fucose sufficient antibodies. These differences in binding affinities resulted in altered A/I ratios that were most pronounced for IgG2b (Figure 5 and Table 2) and translated into significantly enhanced *in vivo* activity for IgG2b. This selective effect of de-fucosylation on FcR binding further illustrates the specificity of IgG subclasses in their interactions with individual FcRs and the predictive value of the A/I ratio in determining *in vivo* activity.

### Example 4

Modified Antibody With A Lower Amount Of Sialic Acid The role of sialic acid residues in antibody sugar side chains was investigated by injecting mice with 6A6-IgG1 antibody variants enriched or depleted for sialic acid residues and measuring antibody mediated platelet depletion. Antibodies enriched for sialic acid in their sugar side chains displayed strongly reduced affinity for both the activating FcRIII and inhibitory FcRIIB (Fig. 6). Consistent with this loss in overall affinity for Fc-receptors and the low A/I ratio of 0.18, this antibody had a severely impaired *in vivo* activity and mediated only minimal platelet depletion (Figure 6).

### Example 5

### Human Antibodies Display Differential A/I Ratios

To investigate if human antibody isotypes also display differential A/I ratios soluble versions of human Fcγ-receptors were prepared and their affinity for human IgG antibody isotypes was measured by surface plasmon resonance analysis. As shown in Table 3 human FcRs also have differential A/I ratios for individual human IgG antibody isotypes.

**Table 3: A/I ratio of the indicated activating Fc-receptors to the inhibitory FcγRIIB**

| | IIA/IIB | IIIA/IIB |
|---|---|---|
| FITC-IgG1 | 3.14 | 5.48 |
| FITC-IgG1 (-Fucose) | 3.06 | 76 |
| FITC-IgG2 | 4.52 | 0.45 |
| FITC-IgG2 (-Fucose) | 5.31 | 5.67 |

| | | |
|---|---|---|
| Numbers represent the affinity (K_{A}) of the indicated antibody isotypes to the indicated soluble Fey-receptors (sFcγR) as measured by surface plasmon resonance analysis (Nimmerjahn et al., (2005)). A/I is the ratio of the affinity of the indicated activating to the inhibitory receptor FcγRIIB. Each data point represents the mean of five experiments performed in duplicates at different concentrations with a SE below 5%. | | |

The affinity data for FcRI was not included as it has been shown to be not relevant for ADCC reactions. (Kaneko et al., (2005) Science 313(5787):670-3.) Based on the data in this table, a person of the ordinary skill in the art will properly conclude that IgG1 will have a greater activity than IgG2, and further that IgG1 lacking fucose will have greater activity than IgG1 containing fucose. Thus, IgG1 more cytotoxic than IgG2 and that the effect of removing fucose will be relevant to IgG1 activity and not to IgG2 activity.

In order to further investigate the characteristics of binding between different human antibody isotypes and different alleles of the FcγRIIA and FcγRIIIA receptors, affinities of binding between an antibody selected from the group consisting of IGG1, IgG2, IgG3, and IgG4, and the Fcγ receptor selected from the group consisting of FcγRIIA^{131H}, FcγRIIA^{131R}, FcγRIIIA^{158P}, and FcγRIIIA^{158V} were determined by SPR. The binding affinities (Kₐ) are shown in Table 1 above. The A/I ratios obtained from these experiments are summarized in table 1.

In contrast to the single chain inhibitory Fc-receptor, activating FcγRs (with the exception of human FcγRIIA) cannot transmit activating signals in the absence of an accessory chain, the common gamma chain (γ-chain), that carries an ITAM motif required for triggering cell activation. FcγRI, FcγRIII and FcγRIV are dependent on γ-chain expression; thus deletion of this receptor subunit leads to the functional loss of all activating Fc-receptors and several other non-FcR-related proteins such as PIR-A and NK cell cytotoxicity receptors (Moretta et al., Annu Rev Immunol 19, 197-223 (2001); Ravetch, (2003)).

The only IgG isotype that could consistently be assigned to an individual activating Fc-receptor in vivo was IgG1. The deletion of the low affinity receptor FcγRIII abrogates IgG1 mediated effector functions in various models like arthritis, glomerulonephritis, IgG-dependent anaphylaxis, IgG mediated hemolytic anemia and immunothrombocytopenia (Hazenbos et al., Immunity 5, 181-188 (1996); Meyer et al., Blood 92, 3997-4002 (1998); Fossati-Jimack et al., J Exp Med 191, 1293-1302 (2000); Ji et al., Immunity 16, 157-168 (2002); Bruhns et al., Immunity 18, 573-581 (2003); Fuji et at., Kidney Int 64, 1406-1416 (2003); Nimmerjahn et al., (2005)). Under many circumstances, such as host response to viral or bacterial infections (Coutelier et al., J Exp Med 165, 64-69 (1987); Schlageter and Kozel, Infect Immun 58, 1914-1918 (1990); Markine-Gorianyoff and Coutelier, J Virol 76, 432-435 (2002); Taborda et al, J Immunol 170, 3621-3630 (2003)), and antibody-mediated cytotoxicity or antibody-based therapy (Kipps et al., J Exp Med 161, 1-17 (1985); Fossati-Jimack et al., J Exp Med 191, 1293-1302 (2000); Uchida et al., J Exp Med 199, 1659-1669 (2004); Nimmerjahn et al., (2005)) the most potent antibody isotypes are of the IgG2a and IgG2b isotype. Therefore, a thorough understanding of how these isotypes exert their function is essential.

Considering the isotype specificities of the high affinity FcγRI (binding exclusively IgG2a) and the low affinity FcγRIII (binding IgG1, IgG2a, and IgG2b) (reviewed in Ravetch and Kinet, Annu Rev Immunol 9, 457-492 (1991); Hulett and Hogarth, Adv Immunol 57, 1-127 (1994)), these two receptors are likely candidates responsible for IgG2a and IgG2b effector functions. Although there is some suggestion that FcγRI and III might participate in a limited fashion in IgG2a-mediated effector responses (Ioan-Facsinay et al., Immunity 16, 391-402 (2002); Barnes et al., Immunity 16, 379-389, (2002)), the majority of studies concluded that IgG2a and IgG2b triggered effects occur independently of these two receptors, but in a gamma chain dependent manner (Hazenbos et al., Immunity 5, 181-188 (1996) ; Meyer et al., (1998); Fossati-Jimack et al., (2000); Uchida et al., (2004); Nimmerjahn et al., (2005)). Especially in the case of IgG2a these results seem to be surprising as FcγRI shows a high affinity for this isotype (KA: 10⁸-10⁹ M⁻¹). However, the increased affinity allowed this receptor to bind monomeric IgG2a as efficiently as immune complexes (ICs), indicating that newly generated ICs would be expected to have only limited access to FcγRI (Figure 7A).

FcγRIV requires γ chain for its surface expression (Nimmerjahn, (2005)) and, as has been described for other γ-chain dependent Fc-receptors, cross-linking of FcγRIV by immune complexes induces activating signaling pathways leading to sustained calcium flux (reviewed in Ravetch and Bolland, (2001); Nimmerjahn et al., (2005)).

Even if several activating Fc-receptors with the same isotype specificity are present on the same cell, only those Fc-receptors will be engaged that show the optimal affinity for the respective isotype (Figure 7A). Therefore, IgG1 immune complexes will only trigger FcγRIII as it is the only activating Fc-receptor that can bind IgG1 (Takai, (1994); Hazenbos et al., (1996); Meyer et al., (1998); Nimmerjahn et al., (2005)). IgG2a and IgG2b, despite their ability to bind FcγRI (in the case of IgG2a) or FcγRIII (in the case of IgG2a and 2b) will functionally be dependent on FcγRIV, as FcγRI will be occupied by monomeric IgG2a and the low affinity of RIII will not result in productive engagement at normal serum concentration of these isotypes. These same principles also apply for the human system, where it has been shown that human FcγRIIIA has a higher affinity for IgG1 as compared to human FcγRIIA. In addition, the presence of allelic variants which show differential affinities for the specific antibody isotypes further supports this concept (Dijstelbloem et al., Trends Immunol, 22, 510-516 (2001)).

The present invention provides a mechanistic basis for the observed variation in IgG subclass activity in both active and passive vaccination and in the variable pathogenicity of the IgG subclasses in autoimmune conditions. The selective FcγR binding affinities of the IgG subclasses, and not their ability to fix complement, is predictive of the *in vivo* activity for cytotoxic antibodies in models of tumor clearance, platelet and B cell depletion (Uchida et al., J Exp Med 199, 1659-69. (2004); Clynes and Ravetch (1995); Clynes (1998); Samuelsson (2001)). Similarly, the biological consequences of modifications to IgG antibodies are, in turn, dependent on their effects on specific FcR binding affinities that result in changes to the ratio of activation to inhibitory receptor affinities. These considerations will be significant factors in the design of both antibody-based immunotherapeutics and active vaccination protocols to insure either the selective engineering of IgG Fc domains or induction of IgG subclasses with optimal FcγR activation to inhibitory ratios.

All publications cited in the specification, both patent publications and non-patent publications, are indicative of the level of skill of those skilled in the art to which this invention pertains. All these publications are herein fully incorporated by reference to the same extent as if each individual publication were specifically and individually indicated as being incorporated by reference.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the following claims.

Further aspects of the invention:
1. A method of predicting the cytotoxic activity of an antibody or variant thereof comprising:
   determining a binding affinity of the antibody or variant thereof to a Fc activating receptor;
   determining a binding affinity of the antibody or variant thereof to a Fc inhibitory receptor, and
   calculating the ratio of said activating binding affinity to said inhibitory binding affinity,
      wherein the magnitude of said ratio is an indication of the cytotoxic activity of the antibody or variant thereof.
2. A method of selecting a cytotoxic antibody or variant thereof out of a plurality of antibodies comprising:
   comparing the A/I ratios of the plurality of antibodies; and
   selecting the cytotoxic antibody or variant thereof with the greater A/I ratio.
3. A method of selecting one or more antibodies or variants thereof comprising determining the A/I ratio of a plurality of antibodies or variants thereof and selecting those antibodies or variants thereof with a A/I ratio of less than 1.
4. A method of selecting one or more cytotoxic antibodies or variants thereof comprising determining the A/I ratio of a plurality of cytotoxic antibodies or variants thereof and selecting those cytotoxic antibodies or variants thereof with a A/I ratio of 1 or greater.
5. The method of item 4 wherein the A/I ratio of the cytotoxic antibodies or variants thereof is between about 10 to less than 500.
6. The method according to item 2 wherein the cytotoxic antibody is a human cytotoxic antibody.
7. The method according to item 1 wherein an Fc activating receptor is selected from the group consisting of FcγRII, FcγRIII, and an Fc inhibitory receptor is FcγRIIB.
8. The method of item 7, wherein the human FcγRII activating receptor is FcγRIIA or FcγRIIC, wherein the human FcγRIII activating receptor is FcγRIIIA, and wherein the human FcγRII inhibitory receptor is FcγRIIB.
9. The method of item 6 wherein the cytotoxic antibody is a chimeric antibody.
10. The method according to item 6 wherein the cytotoxic antibody is a humanized antibody.
11. The method according to item 2 wherein the A/I ratio is determined by an assay selected from the group consisting of a competition or sandwich ELISA, a radioimmunoassay, a dot blot assay, a fluorescence polarization assay, a scintillation proximity assay, a homogeneous time resolved fluorescence assay, a resonant mirror biosensor analysis, and a surface plasmon resonance analysis.
12. A purified modified antibody comprising an IgG Fc region, the modified antibody having a greater A/I ratio as compared to the unmodified antibody.
13. A purified modified antibody comprising an IgG Fc region, the modified antibody having a lower A/I ratio as compared to the unmodified antibody.
14. The purified modified antibody of item 12 comprising a human IgG1, IgG2, IgG3 or IgG4 Fc region, wherein the modified antibody binds to a human Fc activating receptor selected from the group consisting of FcγRII and FcγRIII, and wherein the modified antibody binds to a human FcγRII inhibitory receptor.
15. The modified antibody of item 14, wherein the modified antibody has an increased cytotoxicity activity in vitro.
16. The purified modified antibody of item 14, wherein the human FcγRII activating receptor is FcγRIIA or FcγRIIC, wherein the human FcγRIII activating receptor is FcγRIIIA, and wherein the human FcγRII inhibitory receptor is FcγRIIB.
17. The purified modified antibody of item 12, wherein the modified antibody has a lower amount of sialic acid compared to an unmodified antibody.
18. The purified modified antibody of item 17, wherein the modified antibody is derived from a naturally occurring antibody source.
19. The purified modified antibody of item 18, wherein the modified antibody has less than 20 percent sialic acid content.
20. The purified modified antibody of item 17, wherein the modified antibody is expressed in a cell line having a protein sialylation deficiency.
21. The purified modified antibody of item 20, wherein the modified antibody has less than 4 percent sialic acid content.
22. The purified modified antibody of item 17, wherein the antibody is modified by treatment with sialidase.
23. The purified modified antibody of item 17, wherein the antibody is purified by affinity chromatography.

## Claims

1. A purified modified antibody comprising an IgG Fc region, the modified antibody having a greater A/I ratio as compared to the unmodified antibody.

2. A purified modified antibody comprising an IgG Fc region, the modified antibody having a lower A/I ratio as compared to the unmodified antibody.

3. The purified modified antibody of claim 1 comprising a human IgG1, IgG2, IgG3 or IgG4 Fc region, wherein the modified antibody binds to a human Fc activating receptor selected from the group consisting of FcγRII and FcγRIII, and wherein the modified antibody binds to a human FcγRII inhibitory receptor.

4. The modified antibody of claim 3, wherein the modified antibody has an increased cytotoxicity activity in vitro.

5. The purified modified antibody of claim 3, wherein the human FcγRII activating receptor is FcγRIIA or FcγRIIC, wherein the human FcγRIII activating receptor is FcγRIIIA, and wherein the human FcγRII inhibitory receptor is FcγRIIB.

6. The purified modified antibody of claim 1, wherein the modified antibody has a lower amount of sialic acid compared to an unmodified antibody.

7. The purified modified antibody of claim 6, wherein the modified antibody is derived from a naturally occurring antibody source.

8. The purified modified antibody of claim 7, wherein the modified antibody has less than 20 percent sialic acid content.

9. The purified modified antibody of claim 6, wherein the modified antibody is expressed in a cell line having a protein sialylation deficiency.

10. The purified modified antibody of claim 9, wherein the modified antibody has less than 4 percent sialic acid content.

11. The purified modified antibody of claim 6, wherein the antibody is modified by treatment with sialidase.

12. The purified modified antibody of claim 6, wherein the antibody is purified by affinity chromatography.
